Europäisches Patentamt
European Patent Office
Office européen des brevets

(11) Veröffentlichungsnummer: **0 300 391**
**A1**

# EUROPÄISCHE PATENTANMELDUNG

(21) Anmeldenummer: 88111471.4

(22) Anmeldetag: 16.07.88

(51) Int. Cl.4: C07D 279/16 , C07D 417/12 , A61K 31/54 , //(C07D417/12, 279:16,295:00)

Patentansprüche für folgende Vertragsstaaten: ES + GR.

(30) Priorität: 23.07.87 DE 3724366

(43) Veröffentlichungstag der Anmeldung: 25.01.89 Patentblatt 89/04

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Oekonomopulos, Raymond, Dr.
Lessingweg 83
D-6200 Wiesbaden(DE)
Erfinder: Henning, Rainer, Dr.
Rotenhofstrasse 31
D-6234 Hattersheim am Main(DE)
Erfinder: Lerch, Ulrich, Dr.
Vorderwart 24
D-6238 Hofheim am Taunus(DE)
Erfinder: Schölkens, Bernward, Dr.
Hölderlinstrasse 62
D-6233 Kelkheim (Taunus)(DE)
Erfinder: Linz, Wolfgang, Dr.
Gundelhardtstrasse 2
D-6233 Kelkheim (Taunus)(DE)

(54) **Benzothiazinon-Derivate, Verfahren zu ihrer Herstellung, sie enthaltende Arzneimittel und deren Verwendung.**

(57) Beschrieben werden Verbindungen I

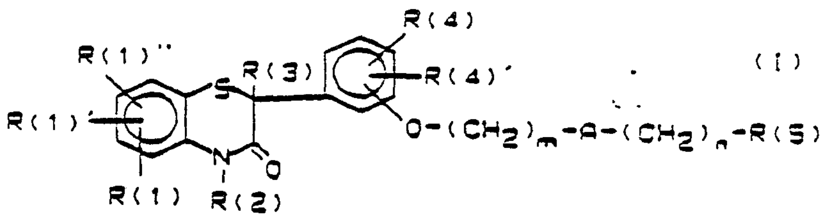

(I)

mit R(1) gleich H, Alkyl, Alkoxy, Hal, $NO_2$, OH, Acetamido, Amino; R(2) gleich H, Alk(en)yl, Phenylalkyl; R(3) gleich H, (Cyclo)-alk(en)yl(alkyl), Phenyl, Phenylalkyl; R(4) gleich H, Alkyl, Alkoxy, Hal, $CF_3$, $NO_2$, OH, Acetamido, Amino; A gleich -C≡C- oder -CH=CH-; R(5) verschiedene Amine; m und n gleich 1-3; sowie ihre Salze. Beschrieben werden auch Herstellungsverfahren.

Verbindungen I sind hervorragende Calciumagonisten oder Calciumantagonisten. Sie haben außerdem Blutzuckerregulierende Wirkung.

EP 0 300 391 A1

## Benzothiazinon-Derivate, Verfahren zu ihrer Herstellung, sie enthaltende Arzneimittel und deren Verwendung

Es ist bekannt, daß Verbindungen, die das Einströmen von Calcium-Ionen in Zellen behindern, als Therapeutika zur Behandlung von verschiedenen Krankheiten, insbesondere des Herz-Kreislauf-Systems beim Menschen und anderen Warmblütern eingesetzt werden können.

Benzothiazinon-Derivate mit calciumantagonistischer Wirkung sind in EP-A-116 368 beschrieben; die dort aufgeführten Verbindungen sind in der 2-Position der Heterocyclus unsubstituiert.

Weiterhin finden sich Benzothiazinon-Derivate mit calciumantagonistischer Wirkung in der EP-A-146 893. Dort werden Verbindungen beschrieben, die am 2-Phenylrest eine basische Ethergruppierung tragen, wobei der basische Stickstoff über eine geradkettige oder verzweigte Alkylkette mit dem Ethersauerstoff verbunden ist.

Wir haben nun überraschend gefunden, daß Verbindungen mit veränderter Seitenkette, abhängig von der Art der Seitenkette, entweder überlegene calciumantagonistische oder calciumagonistische Eigenschaften aufweisen. Diese letztere Eigenschaft macht die entsprechenden Verbindungen unter anderem zur Behandlung der Herzinsuffizienz und der Hypotension geeignet.

Die Erfindung ist daher gerichtet auf Benzothiazinon-Derivate der Formel I, die calciumantagonistische oder calciumagonistische Wirkung aufweisen

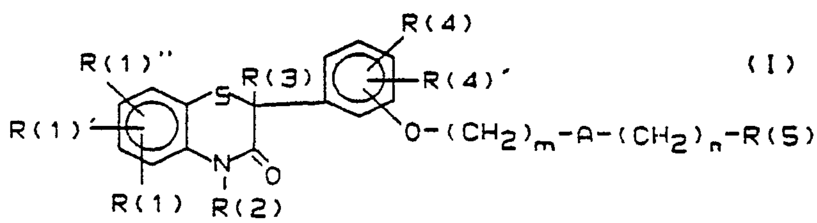

(I)

in welcher Formel I bedeuten:

R(1), R(1)′ und R(1)″, gleich oder verschieden und voneinander unabhängig, Wasserstoff ($C_1$-$C_4$)-Alkyl, ($C_1$-$C_3$)-Alkoxy, F, Cl, Br, $CF_3$, Nitro, Hydroxy, Acetamido oder Amino,

R(2) Wasserstoff, ($C_1$-$C_{10}$)-Alkyl, geradkettig oder verzweigt, ($C_3$-$C_{10}$)-Alkenyl, geradkettig oder verzweigt, Phenyl-($C_1$-$C_4$)-alkyl, wobei der Phenylring unsubstituiert oder durch einen, zwei oder drei Substituenten der Gruppe ($C_1$-$C_4$)-Alkyl, ($C_1$-$C_3$)-Alkoxy, F, Cl, $CF_3$, ($C_1$-$C_2$)-Alkylendioxy oder Nitro substituiert ist,

R(3) Wasserstoff, ($C_1$-$C_{15}$)-Alkyl, geradkettig oder verzweigt, ($C_3$-$C_{15}$)-Alkenyl, geradkettig oder verzweigt, ($C_4$-$C_8$)-Cycloalkyl, ($C_4$-$C_8$)-Cycloalkyl-($C_1$-$C_4$)alkyl, Phenyl oder Phenyl-($C_1$-$C_4$)-alkyl, wobei der Phenylrest unsubstituiert oder durch einen, zwei oder drei Substituenten der Gruppe ($C_1$-$C_4$)-Alkyl, ($C_1$-$C_3$)-Alkoxy, F, Cl, $CF_3$, ($C_1$-$C_2$)-Alkylendioxy oder Nitro substituiert ist,

R(4) und R(4)′, gleich oder verschieden und voneinander unabhängig, Wasserstoff, ($C_1$-$C_4$)-Alkyl, ($C_1$-$C_3$)-Alkoxy, F, Cl, $CF_3$, Nitro, Hydroxy, Acetamido oder Amino,

A eine Gruppe -C ≡ C- oder eine Gruppe -CH = CH-, die cis- oder trans-konfiguriert ist,

R(5) eine der folgenden Gruppen

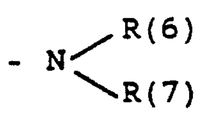
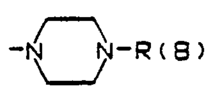
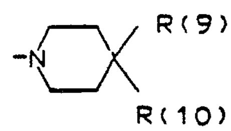

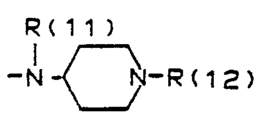

oder

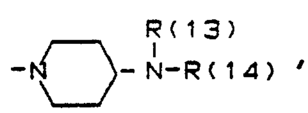

,

worin

R(6) und R(7), gleich oder verschieden, voneinander unabhängig Wasserstoff, ($C_1$-$C_{10}$)-Alkyl, ($C_4$-$C_8$)-Cycloalkyl, ($C_4$-$C_8$)-Cycloalkyl-($C_1$-$C_4$)-alkyl, Pyridyl-($C_1$-$C_4$)-alkyl, Phenyl-($C_1$-$C_6$)-alkyl, Benzhydryl oder

Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind,

R(8) Wasserstoff, ($C_1$-$C_{10}$)-Alkyl, geradkettig oder verzweigt, ($C_1$-$C_8$)-Alkanoyl, Pyridyl, Pyrimidinyl, Phenyl, Phenyl-($C_1$-$C_4$)-alkyl, Phenyl-($C_3$-$C_5$)-alkenyl, Benzhydryl oder Benzylhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind,

R(9) Wasserstoff, ($C_1$-$C_{10}$)-Alkyl, Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert ist,

R(10) Wasserstoff, Hydroxy, oder ($C_1$-$C_4$)-Alkoxy, und

R(11) und R(12) bzw. R(13) und R(14), gleich oder verschieden und voneinander unabhängig Wasserstoff, ($C_1$-$C_{10}$)-Alkyl, geradkettig oder verzweigt, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl, oder Benzoyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind,

m 1, 2 oder 3

n 1, 2 oder 3

bedeuten,

sowie die Salze der Verbindungen der Formel I mit pharmazeutisch akzeptablen Säuren.

Bevorzugt werden die Verbindungen der Formel I, in welcher mindestens einer der Substituenten oder Indices folgende Bedeutung hat:

R(1) und R(1)′, gleich oder verschieden und voneinander unabhängig, Wasserstoff, Methyl, Ethyl, Methoxy, Ethoxy, Fluor, Chlor, $CF_3$, Nitro oder Acetamido,

R(1)″ Wasserstoff,

R(2) Wasserstoff, ($C_1$-$C_6$)-Alkyl, geradkettig oder verzweigt, Allyl, Methallyl, Benzyl, Penethyl, 4-Methoxybenzyl, 3,4-Dimethoxybenzyl, 3,4,5-Trimethoxybenzyl, 3,4-Methylendioxybenzyl,

R(3) Wasserstoff, ($C_1$-$C_{12}$)-Alkyl, geradkettig oder verzweigt, Allyl, Methallyl, ($C_5$-$C_7$)-Cycloalkyl, ($C_5$-$C_7$)-Cycloalkyl-($C_1$-$C_4$)-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)′ Wasserstoff,

A eine Gruppe $-C \equiv C-$ oder eine Gruppe $-CH=CH-$, die cis- oder trans-konfiguriert ist,

R(5) eine der folgenden Gruppen

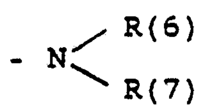

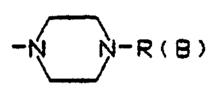

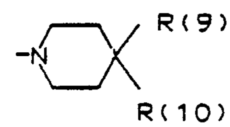

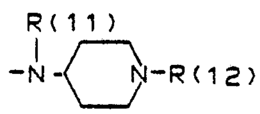

oder

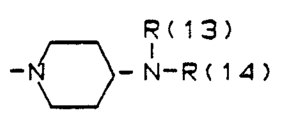

,

worin bedeuten:

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-($C_1$-$C_4$)-alkyl, Benzhydryl oder Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sind, Pyridyl-($C_1$-$C_4$)-alkyl,

R(8) Wasserstoff, ($C_1$-$C_6$)-Alkyl, geradkettig oder verzweigt, ($C_1$-$C_6$)-Alkanoyl, Phenyl, wobei der Phenylrest unsubstituiert oder durch einen oder zwei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist, Phenyl-($C_1$-$C_4$)-alkyl, Phenyl-($C_3$-$C_5$)-alkenyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$-

$C_2$)-Alkylendioxy, F, Cl, $CF_3$ oder Hydroxy substituiert sind,

R(9) Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist

R(10) Wasserstoff, Hydroxy oder Methoxy

R(11), R(12), R(13) und R(14) gleich oder verschieden, Wasserstoff, ($C_1$-$C_8$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste der Gruppe Methyl, Ethyl, Methoxy, Ethoxy, ($C_1$-$C_2$)-Alkylendioxy, F, Cl, $CF_3$ oder Hydroxy substituiert sind,

m 1 oder 2,

n 1 oder 2,

sowie die Salze dieser Verbindungen der Formel I mit pharmazeutisch akzeptablen Säuren.

Besonders bevorzugt werden Verbindungen der Formel I, in welcher mindestens einer der Substituenten oder der Indices bedeuten:

R(1) Wasserstoff, Methyl, Methoxy, Fluor oder Chlor

R(1)′ Wasserstoff oder Methoxy,

R(1)″ Wasserstoff,

R(2) Wasserstoff, Methyl, Ethyl, Propyl, Isopropyl, Butyl, sec. Butyl, Isobutyl, Benzyl, Phenethyl,

R(3) Wasserstoff, ($C_1$-$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)′ Wasserstoff,

A eine Gruppe -C≡C- oder eine Gruppe -CH=CH-, die cis-oder trans-konfiguriert ist,

R(5) eine der folgenden Gruppen

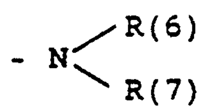

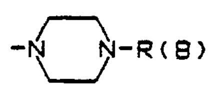

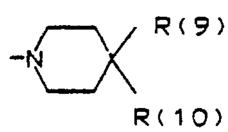

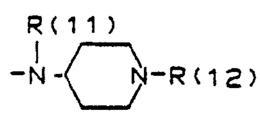

oder

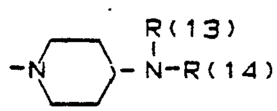

,

worin

R(6) Wasserstoff oder Methyl,

R(7) Phenyl-($C_1$-$C_4$)-alkyl, Benzhydryl oder Benzhydryl-($C_1$-$C_4$)alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist,

R(8) ($C_1$-$C_6$)-Alkyl, geradkettig oder verzweigt, ($C_1$-$C_6$)-Alkanoyl, Phenyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10) Wasserstoff, Hydroxy oder Methoxy,

R(11),R(12), R(13) und R(14), gleich oder verschieden, Wasserstoff, ($C_1$-$C_6$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,

bedeuten,

m und n 1,

sowie die Salze dieser Verbindungen der Formel 1 mit pharmazeutisch akzeptablen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Diese Gemische von Diastereomeren können nach gebräuchlichen Methoden, zum Beispiel selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin Verfahren zur Herstellung von Verbindungen der Formel I, dadurch gekennzeichnet, daß man

a) eine Verbindung der Formel II,

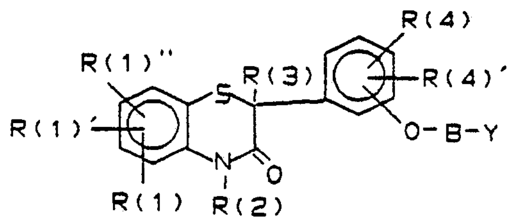

(II)

in welcher R(1), R(1)′, R(1)″, R(2), R(3), R(4) und R(4)′ die gleiche Bedeutung wie in Formel I haben und in welcher B die Reste $-(CH_2)_m-C \equiv C-(CH_2)_n$ oder $-(CH_2)_m-CH=CH-(CH_2)_n$ bedeutet, wobei m und n die gleiche Bedeutung wie in Formel I haben, und in welcher Y eine Abgangsgruppe, die nucleophil verdrängt werden kann, insbesondere ein Chlor-, Brom oder Jodatom, einen Sulfonsäurerest, vorzugsweise einen Methansulfonylrest, einen Benzolsulfonylrest, einen Toluolsulfonylrest oder einen Trifluormethansulfonylrest bedeutet, mit einer der Verbindungen der Formeln IIIa, IIIb, IIIc, IIId oder IIIe

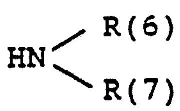

IIIa

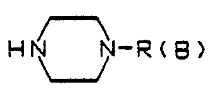

IIIb

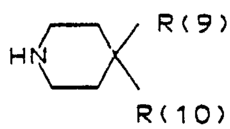

IIIc

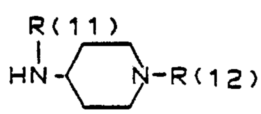

IIId

oder

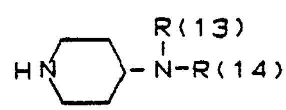

IIIe

,

in welchen R(6), R(7), R(8), R(9), R(10), R(11), R(12), R(13) und R(14) gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol, oder einem niederen Keton, vorzugsweise Aceton oder Methylethylketon, oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160 °C, vorzugsweise zwischen 20 und 120 °C, umsetzt, oder daß man

b) eine Verbindung der Formel IV,

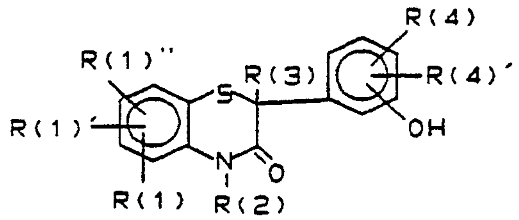

(IV)

in welcher R(1), R(1)′, R(1)″, R(2), R(3), R(4) und R(4)′ die gleiche Bedeutung wie in Formel I haben, mit einer Verbindung der Formel V,

$Z\text{-}(CH_2)_m\text{-}A\text{-}(CH_2)_n\text{-}R(5)$ V,

in welcher Z gleich wie Y in Formel II definiert ist und in welcher R(5), und A die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen -40 und +60°C, vorzugsweise zwischen -10 und -30°C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin, wie Triethylamin, N-Ethylmorpholin, N-Methyldiisopropylamin oder Pyridin, bei einer Temperatur zwischen 0 und 160°C, vorzugsweise zwischen 20 und 120°C, umsetzt, oder daß man

c) eine Verbindung der Formel I, in welcher A die Gruppe -C≡C- darstellt, mit einem Alkalimetall, insbesondere Lithium oder Natrium, in Ammoniak oder einem aliphatischen Amin, insbesondere einem Amin mit 1 bis 6 C-Atomen, bei einer Temperatur zwischen -100°C und dem Siedepunkt des Lösungsmittels, insbesondere bei einer Temperatur zwischen -80°C und -20°C, umsetzt, wobei man eine Verbindung der Formel I erhält, in welcher A eine trans-konfigurierte -CH=CH-Gruppe bedeutet oder daß man

d) eine Verbindung der Formel I, in welcher A die Gruppe -C≡C- darstellt, mit einem Edelmetallkatalysator, insbesondere einem solchen Katalysator, wie er von Lindlar (Helv. Chim. Acta 1952, 35, 446) oder von Cram und Allinger (J. Am. Chem. Soc 1956, 78, 2518) beschrieben worden ist, in einem Kohlenwasserstoff, einem Alkohol oder einem Ester einer niederen Fettsäure mit 1 bis 20 bar Wasserstoffdruck bei einer Temperatur zwischen -20°C und +60°C umsetzt, wobei man eine Verbindung der Formel I erhält, in welcher A eine cis-konfigurierte Doppelbindung bedeutet.

Verbindungen der Formel II erhält man aus Verbindungen der Formel VIII

$Z\text{-}(CH_2)_m\text{-}A\text{-}(CH_2)_m\text{-}Y$ VIII,

worin A und Y die gleiche Bedeutung wie in Formel II haben
und Z eine Abgangsgruppe ist, die nucleophil verdrängt werden kann, wobei Z wie Y definiert ist und gleich Y oder davon verschieden sein kann, unter den unter b) beschriebenen Bedingungen.

Verbindungen der Formel IV sind aus der EP-A-146 893 bekannt.

Alkyl, Alkylen, Alkanoyl, Alkoxy bedeuten, wenn nicht ausdrücklich anders erwähnt, immer gerade oder verzweigte Ketten.

Die erfindungsgemäßen Verbindungen der Formel I sowie ihre pharmakologisch verträglichen Salze weisen, je nach ihrer Struktur, calciumantagonistische oder calciumagonistische Eigenschaften auf. Sie können daher zur Behandlung aller Krankheitszustände, die auf einer Störung des Calciumhaushalts eines Warmblüters beruhen, verwendet werden. Insbesondere sind die calciumantagonistisch wirksamen Verbindungen zur Behandlung des Herz-Kreislauf-Systems bei entsprechenden Beschwerden, z.B. bei verschiedenen Formen der Angina Pectoris, Tachycardie, Herzrhythmusstörungen und Bluthochdruck geeignet. Die calciumagonistisch wirksamen Verbindungen können dagegen z.B. zur Behandlung der Hypotonie, der Herzinsuffizienz verschiedener Genese, aber auch des Diabetes (Typ II) angewandt werden.
Die erfindungsgemäßen Verbindungen und ihre pharmakologisch verträglichen Salze entfalten ihre calciumantagonistischen bzw. calciumagonistischen Wirkungen durch Beeinflussung des Einströmens von Calcium-Ionen in Zellen durch spezifische Kanäle.
Ihre Wirkung auf die Calciumkanäle kann an dem biochemischen Testmodell der Verdrängung von

Tritiummarkiertem Nitrendipin gezeigt werden. Hierbei werden Membranpräparationen, die isolierte Calciumkanäle enthalten, mit der markierten Substanz beladen. Nach Inkubation mit der Testsubstanz wird die freigesetzte Radioaktivität bzw. die an der Membran verbleibende Radioaktivität bestimmt. In diesem Modell weisen die erfindungsgemäßen Verbindungen der Formel I $IC_{50}$-Werte von $10^{-6}$ molar bis $10^{-10}$ molar auf. Beispielsweise hat die Verbindung aus Beispiel 2 einen $IC_{50}$-Wert von 1,2 x $10^{-10}$ M, die Verbindung aus Beispiel 3 einen $IC_{50}$-Wert von 4,5 x $10^{-10}$ M.

Ihre calciumantagonistische bzw. calciumagonistische Wirkung kann am Modell der isolierten Kaninchenaorta gezeigt werden. Hierbei werden Aortenringe mit Kaliumchlorid depolarisiert, woraufhin diese kontrahieren. Nach Vorbehandlung mit der Testsubstanz wird bei den calciumantagonistisch wirksamen Verbindungen eine Verminderung der Konzentration beobachtet, bei den calciumagonistisch wirksamen Verbindungen dagegen eine Verstärkung der Konzentration. Beispielsweise führt die Verbindung aus Beispiel 2 in einer Konzentration von $10^{-6}$ M zu einer Verminderung der Konzentration um 32 %. Die Verbindung aus Beispiel 3 führt in einer Konzentration von $10^{-6}$ M zu einer Verstärkung der Kontraktion um 93 %.

Die erfindungsgemäßen Verbindungen sind in einen breiten Dosisbereich wirksam. Die Höhe der verabreichten Dosis beim Warmblüter, insbesondere beim Menschen ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,01 mg, vorzugsweise ab 0,1 mg und bis zu 100 mg, vorzugsweise bis zu 20 mg einer Verbindung der Formel I pro kg Körpergewicht erreicht. Beim Menschen variert die tägliche Dosis zwischen 10 und 800 mg, vorzugsweise 20 bis 500 mg, wobei Einzeldosen von 5 bis 200 mg, vorzugsweise ein bis dreimal täglich, gegeben werden können.

Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z. B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z. B. mittels konventioneller Misch-Granulier- und Dragierverfahren, hergestellt und enthalten 0,1 % bis etwa 75 %, bevorzugt etwa 1 % bis etwa 50 % des Wirkstoffs.

Die im Anschluß folgenden Beispiele sollen die Erfindung erläutern, ohne sie auf diese Beispiele zu begrenzen.

**Beispiel 1**

(R)-2-[2-(4-Chlor-2-butin-1-yloxy)phenyl]-2-isopropyl-4-methyl-2H-1,4-benzothiazin-3(4H)-on

4,1 g (13 mmol) R-2-(2-Hydroxyphenyl)-2-isopropyl-4-methyl-2H-1,4-benzothiazin-3(4H)-on werden in 10 ml 1 N methanolischer KOH gelöst und auf 50° C erwärmt. Innerhalb von 3 h tropft man unter Rühren eine Lösung von 6,4 g (52 mMol) 1,4-Dichlorbutin-2 in 5 ml Methanol zu. Dabei fällt ein weißer Niederschlag aus, der sich teilweise an der Wand absetzt. Das Absinken des pH-Wertes unter 6,5 korrigiert man bei der weiteren Reaktion durch mehrmalige Zugabe von 1 N methanolischer KOH. Die Reaktion wird dünnschichtchromatographisch kontrolliert (Kieselgel Si 60; mobile Phase : Cyclohexan/Essigester 1 : 1) und nach ca. 6 h Gesamtreaktionszeit abgebrochen.
Der Niederschlag wird abgesaugt, mit Methanol gewaschen und im Hochvakuum getrocknet. Auf diese Weise werden 3,1 g (59 %) reines Produkt isoliert (Schmp. 136° C). Durch Aufarbeiten der Mutterlauge läßt sich die Produktionsausbeute auf insgesamt 90 % erhöhen.

**Beispiel 2**

(R)-2-Isopropyl-4-methyl-2-[2-[4-[4-(3,4,5-trimethoxyphenethyl)-1-piperazinyl]-2-butin-1-yloxy] phenyl]-2H-1,4-benzothiazin-3(4H)-on-Dihydrochlorid

3,1 g (7,8 mMol) der Verbindung aus Beispiel 1 mischt man mit 7 g (20 mMol) 3,4,5-Trimethoxyphenethyl-piperazin und 10 g Kaliumcarbonat (fein gemörsert) und fügt 30 ml Isopropanol zu. Bei ca. 90 °C läßt man 15 h reagieren und trennt nach Abkühlen des Salz ab. Das dunkelbraune Filtrat wird zu einen zähen Öl eingedampft (DC-Kontrolle Kieselgel Si 60; mobile Phase : Dichlormethan/Methanol 20 :1).

Das Rohprodukt wird in Aceton gelöst und mit 5 ml ethanolischer HCl (2,5 N) versetzt. Eine ausfallende Verunreinigung wird abgetrennt und das erneut eingedampfte Produkt auf einer Lobar®Si 60-Säule gereinigt (mobile Phase: Dichlormethan/Methanol 20 : 1). (Auswaage: 3,6 g ≙ 51 %; Schmp. 166 °C; Zers.).

**Beispiel 3**

(R)-2-Isopropyl-4-methyl-2-[2-[4-[4-(3,4,5-trimethoxyphenethyl)-1-piperazinyl]-2-cis-buten-1-yloxy] phenyl]-2H-1,4-benzothiazin-3(4H)-on-Dihydrochlorid

1,44 g (2 mMol) der Verbindung aus Beispiel 2 werden in 10 ml Methanol gelöst und über Lindlarkatalysator, schwarz (Fluka, 62 145) unter Kontrolle des Wasserstoffverbrauches hydriert. Nach Aufnahme der berechneten Wasserstoffmenge wird der Katalysator abgesaugt und das Reaktionsprodukt dünnschichtchromatographisch untersucht (DC auf Kieselgel Si 60; mobile Phase : a) n-Butylacetat/Isopropanol/Wasser/Ammoniaklösung 30:50:15:5; b) Chloroform/Cyclohexan/Eisessig/Ethanol 9:9:1:1).

Das Rohprodukt wird über eine Lobar®Si 60-Säule mit Methylenchlorid/Methanol (20:1) als mobiler Phase gereinigt (Auswaage: 420 mg ≙ 29 %, Schmp. 165 °C; Zers.).

**Ansprüche**

1. Verbindung I der Formel

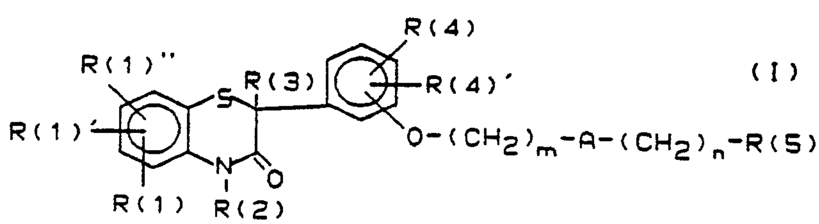

(I)

in welcher Formel I bedeuten:

R(1), R(1)′ und R(1)″, gleich oder verschieden und voneinander unabhängig, Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)′, gleich oder verschieden und voneinander unabhängig, Wasserstoff, $(C_1-C_4)$-Alkyl, $(C_1-C_3)$-

Alkoxy, F, Cl, $CF_3$, Nitro, Hydroxy, Acetamido oder Amino,
A eine Gruppe -C ≡ C- oder eine Gruppe -CH = CH-, die cis- oder trans-konfiguriert ist,
R(5) eine der folgenden Gruppen

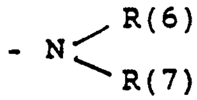 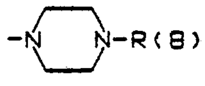 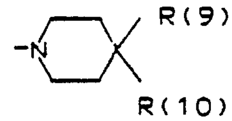

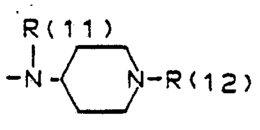 oder 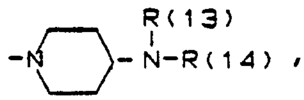 ,

worin
R(6) und R(7), gleich oder verschieden, voneinander unabhängig Wasserstoff, $(C_1$-$C_{10})$-Alkyl, $(C_4$-$C_8)$-Cycloalkyl, $(C_4$-$C_8)$-Cycloalkyl-$(C_1$-$C_4)$-alkyl, Pyridyl-$(C_1$-$C_4)$-alkyl, Phenyl-$(C_1$-$C_6)$-alkyl, Benzhydryl oder Benzhydryl-$(C_1$-$C_4)$-alkyl, wobei die Phenylreste jeweils unsubstituiert oder durch einen, zwei oder drei Reste aus der Gruppe $(C_1$-$C_4)$-Alkyl, $(C_1$-$C_4)$-Alkoxy, $(C_1$-$C_2)$-Alkylendioxy, F, Cl, Br, $CF_3$ oder Hydroxy substituiert sind,
R(8) Wasserstoff, $(C_1$-$C_{10})$-Alkyl, geradkettig oder verzweigt, $(C_1$-$C_8)$-Alkanoyl, Pyridyl, Pyrimidinyl, Phenyl, Phenyl-$(C_1$-$C_4)$-alkyl, Phenyl-$(C_3$-$C_5)$-alkenyl, Benzhydryl oder Benzylhydryl-$(C_1$-$C_4)$-alkyl, Phenyl-$(C_1$-$C_4)$-alkanoyl oder Benzoyl, wobei die Phenylreste jeweils unsubstituiert oder durch einen, zwei oder drei Reste aus der Gruppe $(C_1$-$C_4)$-Alkyl, $(C_1$-$C_4)$-Alkoxy, $(C_1$-$C_2)$-Alkylendioxy, F, Cl, Br, $CF_3$ oder Hydroxy substituiert sind,
R(9) Wasserstoff, $(C_1$-$C_{10})$-Alkyl, Phenyl, Phenyl-$(C_1$-$C_4)$-alkyl, wobei der Phenylrest jeweils unsubstituiert oder durch einen, zwei oder drei Reste aus der Gruppe $(C_1$-$C_4)$-Alkyl, $(C_1$-$C_4)$-Alkoxy, $(C_1$-$C_2)$-Alkylendioxy, F, Cl, Br, $CF_3$ oder Hydroxy substituiert ist,
R(10) Wasserstoff, Hydroxy, oder $(C_1$-$C_4)$-Alkoxy, und
R(11) und R(12) bzw. R(13) und R(14), gleich oder verschieden und voneinander unabhängig Wasserstoff, $(C_1$-$C_{10})$-Alkyl, geradkettig oder verzweigt, $(C_1$-$C_6)$-Alkanoyl, Phenyl-$(C_1$-$C_4)$-alkyl, Benzhydryl oder Benzhydryl-$(C_1$-$C_4)$-alkyl, Phenyl-$(C_1$-$C_4)$-alkanoyl, oder Benzoyl, wobei die Phenylreste jeweils unsubstituiert oder durch einen, zwei oder drei Reste aus der Gruppe $(C_1$-$C_4)$-Alkyl, $(C_1$-$C_4)$-Alkoxy, $(C_1$-$C_2)$-Alkylendioxy, F, Cl, Br, $CF_3$ oder Hydroxy substituiert sind,
m 1, 2 oder 3
n 1, 2 oder 3
bedeuten,
sowie die Salze der Verbindungen der Formel I mit pharmazeutisch akzeptablen Säuren.

2. Verbindung I nach Anspruch 1, dadurch gekennzeichnet, daß mindestens einer der Substituenten oder Indices folgende Bedeutung hat:
R(1) und R(1)′, gleich oder verschieden und voneinander unabhängig, Wasserstoff, Methyl, Ethyl, Methoxy, Ethoxy, Fluor, Chlor, $CF_3$, Nitro oder Acetamido, R(1)″ Wasserstoff,
R(2) Wasserstoff, $(C_1$-$C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,
R(3) Wasserstoff, $(C_1$-$C_{12})$-Alkyl, geradkettig oder verzweigt, Allyl, Methallyl, $(C_5$-$C_7)$-Cycloalkyl, $(C_5$-$C_7)$-Cycloalkyl-$(C_1$-$C_4)$-alkyl, Benzyl, Methylbenzyl, Fluorbenzyl, Methoxybenzyl, Dimethoxybenzyl, Phenylethyl,
R(4) Wasserstoff, Methyl, Methoxy, Ethoxy, Chlor, Nitro, Hydroxy, Acetamido oder Amino,
R(4)′ Wasserstoff,
A eine Gruppe -C≡C- oder eine Gruppe -CH = CH-, die cis- oder trans-konfiguriert ist,
R(5) eine der folgenden Gruppen

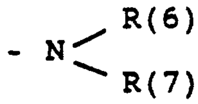 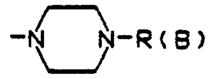 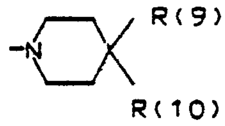

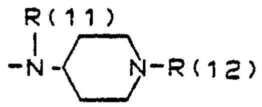 oder 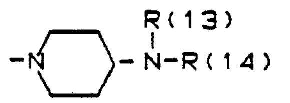 ,

worin bedeuten:

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1-C_4)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert sind, Pyridyl-$(C_1-C_4)$-alkyl,

R(8) Wasserstoff, $(C_1-C_6)$-Alkyl, geradkettig oder verzweigt, $(C_1-C_6)$-Alkanoyl, Phenyl, wobei der Phenylrest unsubstituiert oder durch einen oder zwei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Phenyl-$(C_1-C_4)$-alkyl, Phenyl-$(C_3-C_5)$-alkenyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Ethyl, Methoxy, Ethoxy, $(C_1-C_2)$-Alkylendioxy, F, Cl, $CF_3$ oder Hydroxy substituiert sind,

R(9) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden, Wasserstoff, $(C_1-C_8)$-Alkyl, $(C_1-C_5)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste der Gruppe Methyl, Ethyl, Methoxy, Ethoxy, $(C_1-C_2)$-Alkylendioxy, F, Cl, $CF_3$ oder Hydroxy substituiert sind,

m 1 oder 2,

n 1 oder 2

sowie die Salze dieser Verbindungen der Formel I mit pharmazeutisch akzeptablen Säuren.

3. Verbindung I nach Anspruch 1, dadurch gekennzeichnet, daß mindestens einer der Substituenten oder der Indices bedeuten:

R(1) Wasserstoff, Methyl, Methoxy, Fluor oder Chlor,

R(1)′ Wasserstoff oder Methoxy,

R(1)″ Wasserstoff,

R(2) Wasserstoff, Methyl, Ethyl, Propyl, Isopropyl, Butyl, sec. Butyl, Isobutyl, Benzyl, Phenethyl,

R(3) Wasserstoff, $(C_1-C_{12})$-Alkyl, geradkettig oder verzweigt; Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)′ Wasserstoff,

A eine Gruppe -C≡C- oder eine Gruppe -CH=CH-, die cis-oder trans-konfiguriert ist,

R(5) eine der folgenden Gruppen

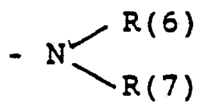
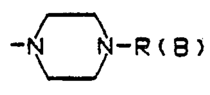
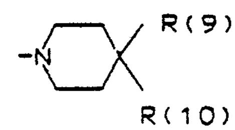

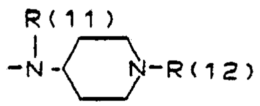 oder 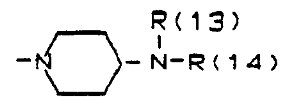 ,

worin

R(6) Wasserstoff oder Methyl,

R(7) Phenyl-($C_1$-$C_4$)-alkyl, Benzhydryl oder Benzhydryl-($C_1$-$C_4$)-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist,

R(8) ($C_1$-$C_6$)-Alkyl, geradkettig oder verzweigt, ($C_1$-$C_6$)-Alkanoyl, Phenyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10) Wasserstoff, Hydroxy oder Methoxy,

R(11),R(12), R(13) und R(14), gleich oder verschieden, Wasserstoff, ($C_1$-$C_6$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,

bedeuten,

m und n 1,

sowie die Salze dieser Verbindungen der Formel I mit pharmazeutisch akzeptablen Säuren.

4. Verfahren zum Herstellen einer Verbindung I nach Anspruch 1, dadurch gekennzeichnet, daß man

a) eine Verbindung der Formel II,

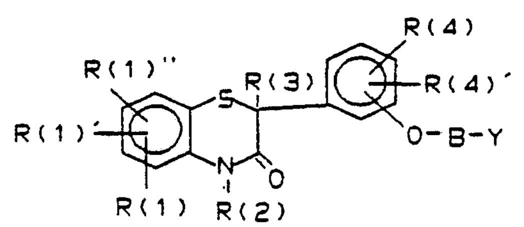

(II)

in welcher R(1), R(1)', R(1)'', R(2), R(3), R(4) und R(4)' die gleiche Bedeutung wie in Formel I haben und in welcher B die Reste $-(CH_2)_m-C{\equiv}C-(CH_2)_n$ oder $-(CH_2)_m-CH=CH-(CH_2)_n$ bedeutet, wobei m und n die gleiche Bedeutung wie in Formel I haben, und in welcher Y eine Abgangsgruppe, die nucleophil verdrängt werden kann, mit einer der Verbindungen der Formeln IIIa, IIIb, IIIc, IIId oder IIIe

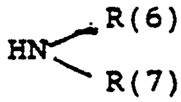
IIIa

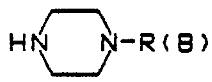
IIIb

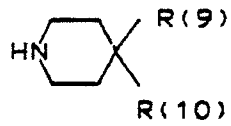
IIIc

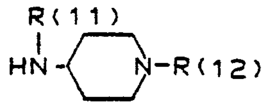
IIId

oder

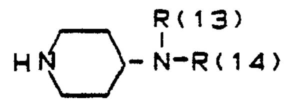
IIIe

,

in welchen R(6), R(7), R(8), R(9), R(10), R(11), R(12), R(13) und R(14) gleiche Bedeutung wie in Formel I haben, unter Bedingungen einer nucleophilen Substitution mit oder ohne Gegenwart einer Hilfsbase zum Abfangen der sich bildenden Säure bei einer Temperatur zwischen 0 und 160° C, umsetzt, oder daß man

b) eine Verbindung der Formel IV,

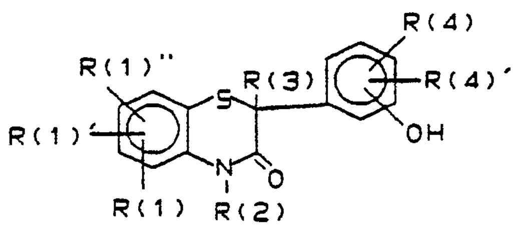
(IV)

in welcher R(1), R(1)′, R(1)″, R(2), R(3), R(4) und R(4)′ die gleiche Bedeutung wie in Formel I haben, mit einer Verbindung der Formel V,

$Z\text{-}(CH_2)_m\text{-}A\text{-}(CH_2)_n\text{-}R(5)$ V,

in welcher Z gleich wie Y in Formel II definiert ist und in welcher R(5), und A die gleiche Bedeutung wie in Formel I haben, entweder in einem polaren aprotischen Lösungsmittel in Gegenwart einer starken Base bei einer Temperatur zwischen -40 und +60° C oder in einem protischen oder aprotischen polaren organischen Lösungsmittel in Gegenwart einer schwachen bis mittelstarken Base bei einer Temperatur zwischen 0 und 160° C umsetzt, oder daß man

c) eine Verbindung der Formel I, in welcher A die Gruppe -C≡C- darstellt, mit einem Alkalimetall in Ammoniak oder einem aliphatischen Amin bei einer Temperatur zwischen -100° C und dem Siedepunkt des Lösungsmittels umsetzt, wobei man eine Verbindung der Formel I erhält, in welcher A eine trans-konfigurierte -CH=CH-Gruppe bedeutet oder daß man

d) eine Verbindung der Formel I, in welcher A die Gruppe -C≡C- darstellt, mit einem Edelmetallkatalysator in einem Kohlenwasserstoff, einem Alkohol oder einem Ester einer niederen Fettsäure mit 1 bis 20 bar Wasserstoffdruck bei einer Temperatur zwischen -20° C und +60° C umsetzt, wobei man eine Verbindung der Formel I erhält, in welcher A eine cis-konfigurierte Doppelbindung bedeutet.

5. Mittel zur Behandlung von Erkrankungen des Herz-Kreislauf-Systems oder von Störungen des Blutzuckerhaushaltes, welches mindestens eine Verbindung I nach Anspruch 1 und übliche Zusatzstoffe enthält.

6. Verwendung einer Verbindung I nach Anspruch 1 zur Behandlung von Erkrankungen des Herz-Kreislaufsystems oder von Störungen des Blutzuckerhaushaltes.

7. Verwendung einer Verbindung I nach Anspruch 1 zur Herstellung eines Medikaments zur Behandlung von Erkrankungen des Herz-Kreislaufsystems oder von Störungen des Blutzuckerhaushaltes.

Patentansprüche fur die folgenden Vertragsstaaten: ES, GR

1. Verfahren zum Herstellen einer Verbindung I

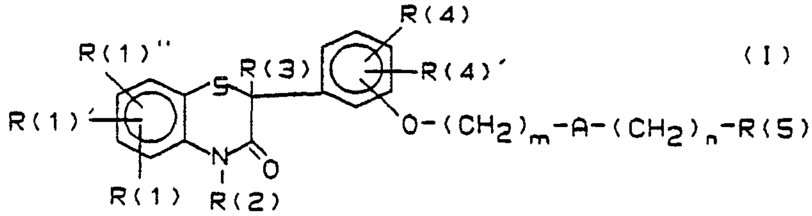

(I)

in welcher Formel I bedeuten:

R(1), R(1)′ und R(1)″, gleich oder verschieden und voneinander unabhängig, Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)′, gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A eine Gruppe -C ≡ C- oder eine Gruppe -CH = CH-, die cis- oder trans-konfiguriert ist,

R(5) eine der folgenden Gruppen

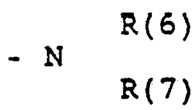 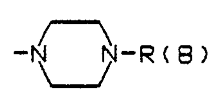 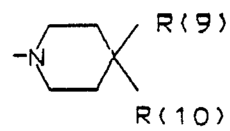

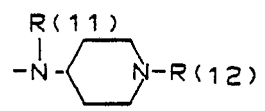 oder 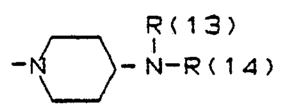 ,

worin

R(6) und R(7), gleich oder verschieden, voneinander unabhängig Wasserstoff, $(C_1-C_{10})$-Alkyl, $(C_4-C_8)$-Cycloalkyl, $(C_4-C_8)$-Cycloalkyl-$(C_1-C_4)$-alkyl, Pyridyl-$(C_1-C_4)$-alkyl, Phenyl-$(C_1-C_6)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(8) Wasserstoff, $(C_1-C_{10})$-Alkyl, geradkettig oder verzweigt, $(C_1-C_8)$-Alkanoyl, Pyridyl, Pyrimidinyl, Phenyl, Phenyl-$(C_1-C_4)$-alkyl, Phenyl-$(C_3-C_5)$-alkenyl, Benzhydryl oder Benzylhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(9) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert ist,

R(10) Wasserstoff, Hydroxy, oder $(C_1-C_4)$-Alkoxy, und

R(11) und R(12) bzw. R(13) und R(14), gleich oder verschieden und voneinander unabhängig Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

m 1, 2 oder 3
n 1, 2 oder 3
bedeuten,
sowie von Salzen der Verbindungen der Formel I mit pharmazeutisch akzeptablen Säuren, dadurch gekennzeichnet, daß man

a) eine Verbindung der Formel II,

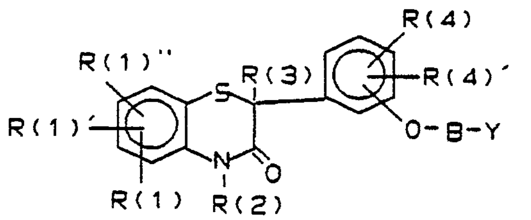

(II)

in welcher R(1), R(1)', R(1)'', R(2), R(3), R(4) und R(4)' die gleiche Bedeutung wie in Formel I haben und in welcher B die Reste $-(CH_2)_m-C{\equiv}C-(CH_2)_n$ oder $-(CH_2)_m-CH{=}CH-(CH_2)_n$ bedeutet, wobei m und n die gleiche Bedeutung wie in Formel I haben, und in welcher Y eine Abgangsgruppe, die nucleophil verdrängt werden kann, mit einer der Verbindungen der Formeln IIIa, IIIb, IIIc, IIId oder IIIe

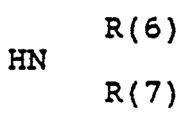

IIIa

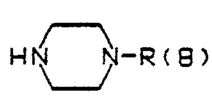

IIIb

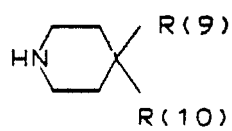

IIIc

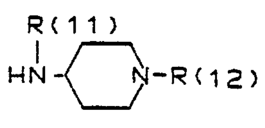

IIId

oder

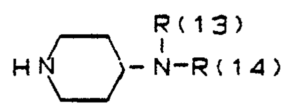

IIIe

,

in welchen R(6), R(7), R(8), R(9), R(10), R(11), R(12), R(13) und R(14) gleiche Bedeutung wie in Formel I haben, unter Bedingungen einer nucleophilen Substitution mit oder ohne Gegenwart einer Hilfsbase zum Abfangen der sich bildenden Säure bei einer Temperatur zwischen 0 und 160 °C, umsetzt, oder daß man

b) eine Verbindung der Formel IV,

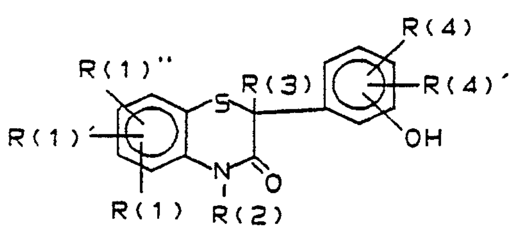

(IV)

in welcher R(1), R(1)', R(1)'', R(2), R(3), R(4) und R(4)' die gleiche Bedeutung wie in Formel I haben, mit einer Verbindung der Formel V,

$Z-(CH_2)_m-A-(CH_2)_n-R(5)$ V,

in welcher Z gleich wie Y in Formel II definiert ist und in welcher R(5), und A die gleiche Bedeutung wie in Formel I haben, entweder in einem polaren aprotischen Lösungsmittel in Gegenwart einer starken Base bei

einer Temperatur zwischen -40 und +60° C oder in einem protischen oder aprotischen polaren organischen Lösungsmittel in Gegenwart einer schwachen bis mittelstarken Base bei einer Temperatur zwischen 0 und 160° C umsetzt, oder daß man

c) eine Verbindung der Formel I, in welcher A die Gruppe -C≡C- darstellt, mit einem Alkalimetall in Ammoniak oder einem aliphatischen Amin bei einer Temperatur zwischen -100° C und dem Siedepunkt des Lösungsmittels umsetzt, wobei man eine Verbindung der Formel I erhält, in welcher A eine trans-konfigurierte -CH=CH- Gruppe bedeutet oder daß man

d) eine Verbindung der Formel I, in welcher A die Gruppe -C≡C- darstellt, mit einem Edelmetallkatalysator in einem Kohlenwasserstoff, einem Alkohol oder einem Ester einer niederen Fettsäure mit 1 bis 20 bar Wasserstoffdruck bei einer Temperatur zwischen -20° C und +60° C umsetzt, wobei man eine Verbindung der Formel I erhält, in welcher A eine cis-konfigurierte Doppelbindung bedeutet.

2. Verfahren zum Herstellen einer Verbindung I nach Anspruch 1, dadurch gekennzeichnet, daß mindestens einer der Substituenten oder Indices folgende Bedeutung hat:

R(1) und R(1)′, gleich oder verschieden und voneinander unabhängig, Wasserstoff, Methyl, Ethyl, Methoxy, Ethoxy, Fluor, Chlor, $CF_3$, Nitro oder Acetamido,

R(1)″ Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$-Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)′ Wasserstoff,

A eine Gruppe -C≡C- oder eine Gruppe -CH=CH-, die cis- oder trans-konfiguriert ist,

R(5) eine der folgenden Gruppen

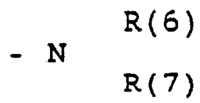

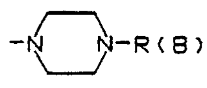

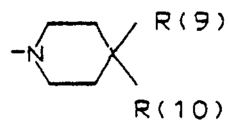

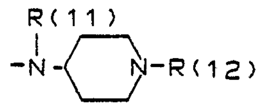

oder

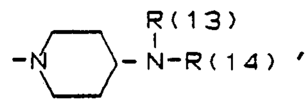

worin bedeuten:

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1-C_4)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert sind, Pyridyl-$(C_1-C_4)$-alkyl,

R(8) Wasserstoff, $(C_1-C_6)$-Alkyl, geradkettig oder verzweigt, $(C_1-C_6)$-Alkanoyl, Phenyl, wobei der Phenylrest unsubstituiert oder durch einen oder zwei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist, Phenyl-$(C_1-C_4)$-alkyl, Phenyl-$(C_3-C_5)$-alkenyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Ethyl, Methoxy, Ethoxy, $(C_1-C_2)$-Alkylendioxy, F, Cl, $CF_3$ oder Hydroxy substituiert sind,

R(9) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden, Wasserstoff, $(C_1-C_8)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$- alkanoyl oder Benzoyl, wobei die Phenylreste jeweils unsubstituiert oder durch einen, zwei oder drei Reste der Gruppe Methyl, Ethyl, Methoxy, Ethoxy, $(C_1-C_2)$-Alkylendioxy, F, Cl, $CF_3$ oder Hydroxy substituiert sind,

m 1 oder 2.
n 1 oder 2,

3. Verfahren zur Herstellung einer Verbindung I nach Anspruch 1, dadurch gekennzeichnet, daß mindestens einer der Substituenten oder der Indices bedeuten:
R(1) Wasserstoff, Methyl, Methoxy, Fluor oder Chlor,
R(1)′ Wasserstoff oder Methoxy,
R(1)″ Wasserstoff,
R(2) Wasserstoff, Methyl, Ethyl, Propyl, Isopropyl, Butyl, sec. Butyl, Isobutyl, Benzyl, Phenethyl,
R(3) Wasserstoff, $(C_1-C_{12})$-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,
R(4) Wasserstoff, Methoxy, Methyl, Chlor, Nitro oder Hydroxy,
R(4)′ Wasserstoff,
A eine Gruppe -C≡C- oder eine Gruppe -CH=CH-, die cis-oder trans-konfiguriert ist,
R(5) eine der folgenden Gruppen

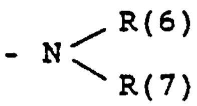
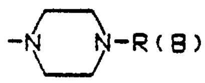
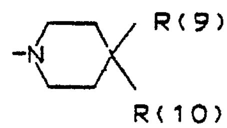

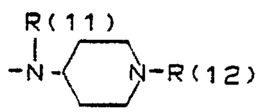
oder
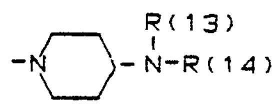
,

worin
R(6) Wasserstoff oder Methyl,
R(7) Phenyl-$(C_1-C_4)$-alkyl, Benzhydryl oder Benzhydryl-$(C_1-C_4)$alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist,
R(8) $(C_1-C_6)$-Alkyl, geradkettig oder verzweigt, $(C_1-C_6)$-Alkanoyl, Phenyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,
R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,
R(10) Wasserstoff, Hydroxy oder Methoxy,
R(11),R(12), R(13) und R(14), gleich oder verschieden, Wasserstoff, $(C_1-C_6)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,
bedeuten,
m und n 1.

4. Verwendung einer Verbindung I nach Anspruch 1 zur Behandlung von Erkrankungen des Herz-Kreislaufsystems oder von Störungen des Blutzuckerhaushaltes.

5. Verwendung einer Verbindung I nach Anspruch 1 zur Herstellung eines Medikaments zur Behandlung von Erkrankungen des Herz-Kreislaufsystems oder von Störungen des Blutzuckerhaushaltes.

Europäisches Patentamt

**EUROPÄISCHER TEILRECHERCHENBERICHT**

Nummer der Anmeldung

EP 88111471.4

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 104, Nr. 13, 31. März 1986, Columbus, Ohio, USA<br>SANTEN PHARMACEUTICAL CO., LTD. "Benzothiazines"<br>Seite 731, Spalte 1, Zusammenfassung Nr. 109 666p<br>& Jpn. Kokai Tokkyo Koho Jp 60-166 674 (85-166 674)<br>---- | 1,4,5,7 | |
| | | | RECHERCHIERTE SACHGEBIETE (Int. Cl.4) |

EPA Form 1505.3 06.78

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88111471.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | DE - A1 - 3 614 363 (HOECHST AG)<br>* Ansprüche 1,4-6; Seite 11, Zeilen 65-68 *<br>-- | 1,4,5,7 | C 07 D 279/16<br>C 07 D 417/12<br>A 61 K 31/54<br>(C 07 D 417/12,<br>C 07 D 279:16,<br>C 07 D 295:00) |
| A,P | DE - A1 - 3 614 335 (HOECHST AG)<br>* Ansprüche 1,4-6; Seite 10, Zeilen 60-63 *<br>-- | 1,4,5,7 | |
| A | WO - A1 - 86/05 490 (SANTEN PHARMACEUTICAL CO., LTD.)<br>* Zusammenfassung *<br>-- | 1,4,5,7 | |

RECHERCHIERTE SACHGEBIETE (Int. Cl.4)

C 07 D 279/00
C 07 D 417/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7
Unvollständig recherchierte Patentansprüche: -
Nicht recherchierte Patentansprüche: 6
Grund für die Beschränkung der Recherche:

Verfahren zur therapeutischen Behandlung des menschlichen oder tierischen Körpers - Art. 52(4), EPÜ

| Recherchenort | Abschlußdatum der Recherche | Prüfer |
|---|---|---|
| WIEN | 28-10-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82